# EUROPEAN PATENT APPLICATION

(11) **EP 3 257 512 A1**
(43) Date of publication of application: **20.12.2017**
(21) Application number: 16749300.6
(22) Date of filing: 12.02.2016
(51) Int. Cl.: A61K 31/4709, A61K 9/06, A61K 9/08, A61K 9/10, A61K 9/70, A61K 47/10, A61K 47/38, A61P 17/00, A61P 17/02, A61P 17/10, A61P 31/04

(54) **EXTERNAL PREPARATION COMPRISING PYRIDONECARBOXYLIC ACID DERIVATIVE**

(30) Priority: 13.02.2015 JP 2015025970
(71) Applicant: Maruho Co., Ltd., Osaka 531-0071 (JP)
(72) Inventor: FUJIKAWA, Akira, osaka-shi Osaka 531-0071 (JP); YAMADA, Hiroshi, osaka-shi Osaka 531-0071 (JP)
(74) Representative: Hoffmann Eitle
(86) International application number: PCT/JP2016/054046
(87) International publication number: WO 2016/129657

(57) **Abstract**

The purpose of the present invention is to provide an external preparation which exerts a therapeutic and/or prophylactic effect on infectious diseases in the field of dermatology such as acne accompanied by suppurative inflammation or epidermal skin infectious diseases when administered once a day mainly to a human patient. As an example of the external preparation according to the present invention, an external preparation, which is characterized by comprising as an active ingredient 1-cyclopropyl-8-methyl-7-[5-methyl-6-(methylamino)-3-pyridyl]-4-oxo-1,4-dihydro-3-quinolincarboxylic acid and/or a pharmaceutically acceptable salt thereof and exerting a therapeutic and/or prophylactic effect on infectious diseases in the field of dermatology when administered once a day to a human patient, can be cited.

## Description

### TECHNICAL FIELD

The present invention relates to an external preparation that, with the pyridonecarboxylic acid derivative 1-cyclopropyl-8-methyl-7-[5-methyl-6-(methylamino)-3-pyridyl]-4-oxo-1,4-dihydro-3-quinolinecarboxylic acid and/or a pharmaceutically acceptable salt thereof contained as an active ingredient, exerts a therapeutic and/or preventive effect against dermatological infections such as acne involving suppurative inflammation, and superficial infections of the skin upon being administered to a human patient once daily.

### BACKGROUND ART

Dermatological infections such as acne involving suppurative inflammation, and superficial infections of the skin have multiple causes. However, the major cause of dermatological infections is the proliferation of Propionibacterium acnes, staphylococcus, and other gram-positive anaerobic bacteria in the sebaceous follicle.

The common traditional treatment of acne involving suppurative inflammation, and superficial infections of the skin include external use of antibiotics, such as nadifloxacin, for mild to moderate conditions, and use of oral antibiotics, such as minocycline, and roxithromycin, for moderate to severe conditions.

Preparations containing 1-cyclopropyl-8-methyl-7-[5-methyl-6-(methylamino)-3-pyridyl]-4-oxo-1,4-dihydro-3-quinolinecarboxylic acid as an active ingredient have been developed to provide a novel drug as an antibiotic for external use (see, for example, Patent Documents 1 and 2).

For improved therapeutic effect, it is of interest and importance to improve patient compliance. Drugs that are expected to be effective may not exert effect, or, worse, may develop only the side effects if the patient does not comply with medication as intended.

One approach to improving patient compliance is to give directions through a pharmacist. There are also active studies directed to reducing the dose frequency, such as by using a sustained-release preparation.

### CITED REFERENCES

### PATENT DOCUMENT

Patent Document 1: WO99/51588
Patent Document 2: WO2007/015453

### DISCLOSURE OF THE INVENTION

### PROBLEMS TO BE SOLVED BY THE INVENTION

The present invention is intended mainly to provide an external preparation that exerts a therapeutic and/or preventive effect against dermatological infections such as acne involving suppurative inflammation, and superficial infections of the skin upon being administered to a human patient once daily.

### MEANS FOR SOLVING THE PROBLEMS

The present inventors conducted intensive studies, and found that the foregoing object can be achieved by using 1-cyclopropyl-8-methyl-7-[5-methyl-6-(methylamino)-3-pyridyl]-4-oxo-1,4-dihydro-3-quinolinecarboxylic acid as an active ingredient. The present invention was completed on the basis of this finding.

The present invention includes the following, for example.
(1) An external preparation (hereinafter, referred to as "external preparation of the present invention") that comprises 1-cyclopropyl-8-methyl-7-[5-methyl-6-(methylamino)-3-pyridyl]-4-oxo-1,4-dihydro-3-quinolinecarboxylic acid (hereinafter, referred to as "compound A according to the present invention") and/or a pharmaceutically acceptable salt thereof as an active ingredient, and that exerts a therapeutic and/or preventive effect against dermatological infections upon being administered to a human patient once daily.
(2) The external preparation as described in (1) above, wherein the dermatological infection is an uncomplicated skin and soft tissue infection, a complicated skin and soft tissue infection, or acne involving suppurative inflammation.
(3) The external preparation as described in (2) above, wherein the uncomplicated skin and soft tissue infection is a superficial infection of the skin, or a deep skin infection.
(4) The external preparation as described in (3) above, wherein the superficial infection of the skin is folliculitis, sycosis, purulent periporitis, or contagious impetigo.
(5) The external preparation as described in (2) above, wherein the acne involving suppurative inflammation is acne vulgaris, neonatal acne, or acne conglobata.
(6) The external preparation as described in (1) above, which has a form of an ointment, a gel, a cream, an emulsion, an adhesive tape, or a lotion.
(7) The external preparation as described in (1) above, which has a form of a lotion.
(8) The external preparation as described in (7) above, which contains a lower alcohol, a water-soluble polymer, and a polyalcohol, and has a pH of 9 to 12.
(9) The external preparation as described in (8) above, wherein the lower alcohol is ethanol or isopropanol.
(10) The external preparation as described in (8) above, wherein the water-soluble polymer is a water-soluble cellulose derivative.
(11) The external preparation as described in (10) above, wherein the water-soluble cellulose derivative is hydroxyethyl cellulose.
(12) The external preparation as described in (8) above, wherein the polyalcohol is 1,3-butylene glycol.
(13) The external preparation as described in (8) above, wherein the lower alcohol is ethanol, the water-soluble polymer is a water-soluble cellulose derivative, and the polyalcohol is 1,3-butylene glycol.
(14) The external preparation as described in (13) above, wherein the ethanol is contained in a content of 1 to 20 weight%.

### BRIEF DESCRIPTION OF DRAWINGS

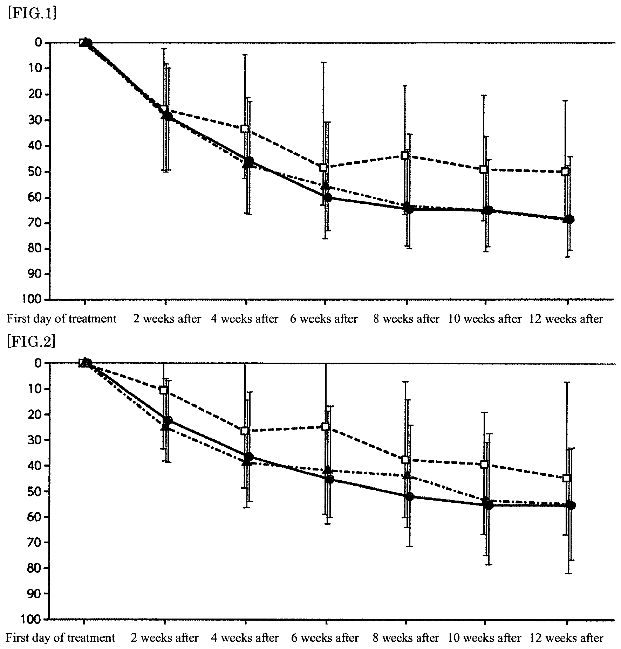

FIG. 1 represents time-course changes in the number of inflammatory lesions due to acne involving suppurative inflammation, in which the vertical axis represents the percentage reduction (%) of the number of inflammatory lesions, the horizontal axis represents evaluation time, the solid circle represents a group that had a test drug administered twice daily, the solid triangle represents a group that had the test drug administered once daily, and the blank square represents a group that had application of a control drug.

FIG. 2 represents time-course changes in the number of inflammatory lesions due to acne involving suppurative inflammation, in which the vertical axis represents the percentage reduction (%) of the number of inflammatory lesions, the horizontal axis represents evaluation time, the solid circle represents a group that had a test drug administered once daily, the solid triangle represents a group that had a comparative drug administered twice daily, and the blank square represents a group that had application of a control drug.

### EMBODIMENTS FOR CARRYING OUT THE INVENTION

The compound A according to the present invention belongs to a group of synthetic antimicrobial compounds of the quinolone family, and exerts its antimicrobial effect by inhibiting the DNA gyrase and the topoisomerase IV involved in DNA replication of bacteria.

The compound A according to the present invention has a wide antimicrobial spectrum, and strong antimicrobial activity against gram-positive bacteria, gram-negative bacteria, anaerobic bacteria, chlamydia, and drug-resistant gram-positive bacteria.

The compound A according to the present invention can be synthesized using the method described in WO99/51588.

Examples of the pharmaceutically acceptable salt of the compound A according to the present invention include commonly known salts of a basic group such as an amino group, and commonly known salts of an acidic group such as a hydroxyl group and a carboxyl group.

Examples of the salts of a basic group include salts with mineral acids such as hydrochloric acid, hydrobromic acid, and sulfuric acid; salts with organic carboxylic acids such as tartaric acid, formic acid, fumaric acid, maleic acid, malic acid, and citric acid; and salts with sulfonic acids such as methanesulfonic acid, benzenesulfonic acid, p-toluenesulfonic acid, mesitylenesulfonic acid, and naphthalenesulfonic acid.

Examples of the salts of an acidic group include salts with alkali metals such as sodium, and potassium; salts with alkali earth metal such as calcium, and magnesium; ammonium salts; and salts with amino acids such as lysine, arginine, and ornithine, or with nitrogen-containing organic bases such as trimethylamine, triethylamine, tributylamine, pyridine, N,N-dimethylaniline, N-methylpiperidine, N-methylmorpholine, diethylamine, dicyclohexylamine, procaine, dibenzylamine, N-benzyl-β-phenethylamine, 1-ephenamine, and N,N'-dibenzylethylenediamine.

With the antimicrobial effect of the compound A according to the present invention contained as an active ingredient, the external preparation of the present invention can be used to treat and/or prevent a wide range of dermatological infections.

The dermatological infections of interest to the external preparation of the present invention are not particularly limited, as long as they are diseases caused in part by bacterial infection. Examples of such diseases include uncomplicated skin and soft tissue infections, complicated skin and soft tissue infections, and acne involving suppurative inflammation.

Examples of the uncomplicated skin and soft tissue infections include superficial infections of the skin, and deep skin infection. Particularly preferred are superficial infections of the skin.

The superficial infections of the skin can be divided into appendage-associated infections and non-appendage-associated infections.

Examples of the appendage-associated infections include folliculitis, sycosis, and purulent periporitis.

Examples of the non-appendage-associated infections include contagious impetigo.

The complicated skin and soft tissue infections also can be divided into appendage-associated infections and non-appendage-associated infections.

Examples of the appendage-associated infections include furuncle, furunculosis, and carbuncle.

Examples of the non-appendage-associated infections include phlegmon, erysipelas, lymphangitis, and lymphadenitis.

The complicated skin and soft tissue infections can be divided into chronic pyoderma, and secondary infections of the skin.

Examples of the chronic pyoderma include infectious atheroma, and hidradenitis suppurativa.

Examples of the secondary infections of the skin include secondary infections such as skin ulcer.

Examples of the acne involving suppurative inflammation include acne vulgaris, neonatal acne, and acne conglobata. Particularly preferred is acne vulgaris.

The external preparation of the present invention can be prepared by appropriately mixing the constituent components using a method well known to a skilled artisan.

The dosage form of the external preparation of the present invention is not particularly limited, and may be, for example, an ointment, a gel, a cream, an emulsion, an adhesive tape, or a lotion. Preferred is a lotion.

The content of the compound A according to the present invention and/or a pharmaceutically acceptable salt thereof in the external preparation of the present invention is not particularly limited, as long as it is an amount that exerts the therapeutic effect. For example, the appropriate content in the preparation is 0.01 to 20 weight%, preferably 0.1 to 5 weight%.

The external preparation of the present invention can exert its therapeutic and/or preventive effect against dermatological infections upon being administered to a human patient once daily.

The dose of the external preparation of the present invention is appropriately selected according to the age, body weight, and symptoms of a patient. Typically, the external preparation of the present invention can exert its drug effect when transdermally administered in an amount of 30 to 2,000 mg/day.

When the dosage form of the external preparation of the present invention is a lotion, the preparation may contain, for example, a lower alcohol, a water-soluble polymer, a polyalcohol, and a pH adjuster, though the constituent components are not particularly limited.

The lower alcohol that may be used in the lotion according to the present invention is not particularly limited, as long as it is a C1 to C3 alcohol, and may be, for example, methanol, ethanol, propanol, or isopropanol. Preferred are ethanol, and isopropanol. Particularly preferred is ethanol.

The appropriate content of lower alcohol is 1 to 20 weight%, preferably 5 to 10 weight% of the total amount of the lotion according to the present invention.

The water-soluble polymer that may be used in the lotion according to the present invention is not particularly limited, as long as it is a water-soluble polymer commonly used for external preparations and cosmetics. Examples include polyoxyethylene polymers such as polyethylene glycol 400, polyethylene glycol 4000, polyethylene glycol 20000, polyethylene glycol 400000, polyethylene glycol 600000, and polyethylene glycol 4000000; a copolymerizable polymer of a polyoxyethylene-polyoxypropylene copolymer; acrylic polymers such as sodium polyacrylate, polyethyl acrylate, and polyacrylamide; and cellulose derivatives such as methyl cellulose, hydrophobized hydroxypropyl methyl cellulose, hydroxyethyl cellulose, and hydroxypropyl cellulose. Preferred are cellulose derivatives, and hydroxyethyl cellulose is more preferred.

These may be used alone or in a combination of two or more. The appropriate content is 0.1 to 5 weight%, preferably 0.7 to 2 weight% of the total amount of the lotion according to the present invention.

The polyalcohol that may be used in the lotion according to the present invention is not particularly limited, as long as it has two or more hydroxyl groups within the molecule, and is one commonly used for external preparations and cosmetics. Examples of such polyalcohols include ethylene glycol, diethylene glycol, triethylene glycol, polyethylene glycol, propylene glycol, dipropylene glycol, hexylene glycol, 1,3-butylene glycol, glycerine, diglycerin, polyglycerin, sorbitol, xylitol, and mannitol. Preferred is 1, 3-butylene glycol.

These may be used alone or in a combination of two or more. The appropriate content is 1 to 30 weight% of the total amount of the lotion according to the present invention.

The pH adjuster that may be used in the lotion according to the present invention is not particularly limited, as long as it can bring the pH to 9 to 12, and is a compound with an additional, buffering capacity. Examples include metal hydroxides such as sodium hydroxide, potassium hydroxide, and lithium hydroxide; hydroxy lower alkylamines such as monoethanolamine, monoisopropanolamine, diethanolamine, diisopropanolamine, triethanolamine, triisopropanolamine, and 2-amino-2-methyl-1,3-propanediol; and metal salts of weak acids, such as sodium bicarbonate, sodium citrate, sodium lactate, disodium hydrogen phosphate, and sodium tartrate. Preferred are metal hydroxides, and metal salts of weak acids. Potassium hydroxide, and sodium bicarbonate are more preferred.

These may be used alone or in a combination of two or more. The appropriate content is 0.01 to 20 weight%, preferably 0.1 to 2 weight% of the total amount of the lotion according to the present invention.

A stabilizer or the like may be additionally mixed with the lotion according to the present invention.

The stabilizer is not particularly limited, and may be, for example, ascorbic acid, sodium edetate, sodium thiosulfate, sodium sulfite, sodium pyrosulfite, sodium nitrite, sodium bisulfite, or photosensitizer 201.

A preservative may be added, as required, though the lotion according to the present invention has preservative effect without a preservative.

### EXAMPLES

The present invention is described below in greater detail using Preparation Examples and Test Examples. The present invention, however, is not limited to the scope of the examples described below.

### Preparation Example 1

A lotion as an external preparation of the present invention was prepared in the following formulation using the method described in WO2007/015453.

**[Table 1]**

| Preparation Example 1 | |
|---|---|
| Formulation | Weight% |
| Compound A | 0.25 |
| Hydroxyethyl cellulose | 1.2 |
| 1,3-Butylene glycol | 15 |
| Ethanol | 5 |
| Potassium hydroxide | 0.265 |
| Sodium bicarbonate | 0.5 |
| Stabilizer | Appropriate amount |
| Purified water | Remainder |
| pH | 10.9 |

### Preparation Example 2

A lotion as an external preparation of the present invention was prepared in the following formulation using the method described in WO2007/015453.

**[Table 2]**

| Preparation Example 2 | |
|---|---|
| Formulation | Weight% |
| Compound A | 0.5 |
| Hydroxyethyl cellulose | 1.2 |
| 1,3-Butylene glycol | 15 |
| Ethanol | 5 |
| Potassium hydroxide | 0.31 |
| Sodium bicarbonate | 0.5 |
| Stabilizer | Appropriate amount |
| Purified water | Remainder |
| pH | 10.9 |

### Preparation Example 3

A lotion as an external preparation of the present invention was prepared in the following formulation using the method described in WO2007/015453.

**[Table 3]**

| Preparation Example 3 | |
|---|---|
| Formulation | Weight% |
| Compound A | 1 |
| Hvdroxvethyl cellulose | 1.2 |
| 1,3-Butylene glycol | 15 |
| Ethanol | 5 |
| Potassium hydroxide | 0.4 |
| Sodium bicarbonate | 0.5 |
| Stabilizer | Appropriate amount |
| Purified water | Remainder |
| pH | 10.9 |

### Preparation Example 4

A lotion as an external preparation of the present invention was prepared in the following formulation using the method described in WO2007/015453.

**[Table 4]**

| Preparation Example 4 | |
|---|---|
| Formulation | Weight% |
| Compound A | 2 |
| Hydroxyethyl cellulose | 1.2 |
| 1,3-Butylene glycol | 15 |
| Ethanol | 5 |
| Potassium hydroxide | 0.58 |
| Sodium bicarbonate | 0.5 |
| Stabilizer | Appropriate amount |
| Purified water | Remainder |
| pH | 10.9 |

### Test Example 1

In order to evaluate the therapeutic effect of the external preparation of the present invention against acne vulgaris, a randomized, parallel-group comparison test was conducted for acne vulgaris patients.

### (1) Subject Patients

Patients, aged 13 or over, involving an inflammatory lesions with 10 or more and less than 30 over the face were chosen as subjects.

Individuals that fell into any of the following categories were excluded even if the foregoing conditions were met.
1) Patients involving acne other than acne vulgaris on face (e.g., acne conglobata, steroidacne, necrotic acne, occupational acne)
2) Patients having complications on face that are considered to affect the evaluation of the test drug (e.g., eczema, atopic dermatitis, rosacea)
3) Patients with clearly visible changes occurring in acne symptoms due to menstrual cycle
4) Patients who had a drug (including common drugs) locally administered to face within 2 weeks before the start of treatment
5) Patients who used any of the following systemically administered drugs (e.g., internally or through an injection to provide a systemic effect) within 4 weeks before the start of treatment
   - Therapeutic drugs for acne (including common drugs, excluding vitamin B2 and B6)
   - Steroids
   - Antibiotics
   - Medicinal agents, including therapeutic agents for acne, that are used outside of Japan, and are considered to affect the efficacy evaluation of the test drug
6) Patients who had either of the following facial treatments within 4 weeks before the start of treatment
   - Chemical peel
   - Laser therapy or phototherapy
7) Patients who used a face scrub within 2 weeks before the start of treatment
8) Patients with a history of hypersensitivity to synthetic antibiotics of the quinolone family
9) Patients with a history of dermal hypersensitivity to external preparations
10) Patients suspected of serious complications through an interview
11) Pregnant patients, and patients wishing to be pregnant or who will be breast feeding during treatment
12) Patients who participated in other test within 6 months (180 days) before the start of treatment
13) Patients who were determined to be inappropriate by a physician conducting the test.

### (2) Test Method

The subject patients were divided into test drug-administered groups (a once daily administered group, and a twice daily administered group), a comparative drug-administered group, and a control drug-administered group. A test drug, a comparative drug, or a control drug was administered for 4 weeks, in the morning and at night.

The preparation prepared in Preparation Example 3 was used as the test drug. A 1% nadifloxacin lotion (a commercially available product, twice daily) was used as the comparative drug. A lotion that did not contain compound A was used as the control drug.

For the once daily test drug-administered group, the control drug was administered in the morning, and the test drug was administered at night.

The face was inspected every week after the treatment was started, and the number of inflammatory lesions was counted.

Here, inflammatory lesion means conditions involving an erythematous papule (a reddish elevated papula measuring no larger than 1 cm in diameter and matching hair follicles), or a pustule (involving occasional tenderness, and including a lesion involving a yellow pus at the top of an erythematous papule, and a lesion appearing yellowish white in color as a whole).

### (3) Evaluation Method

Summary statistics, and a 95% confidence interval were calculated for the percentage reduction of the number of inflammatory lesions in each administered group at the final evaluation time.

### (4) Evaluation Result

As shown in Table 5, the percentage reduction of the number of inflammatory lesions (mean ± standard deviation) at the final evaluation time was higher in the test drug-administered groups and the comparative drug-administered group than in the control drug-administered group.

**[Table 5]**

| Administered group | Twice daily test drug-administered group | Once daily test drug-administered group | Control drug-administered group | Comparative drug-administered group |
|---|---|---|---|---|
| Number of subjects | 44 | 45 | 44 | 45 |
| Mean ± standard deviation | 47.35 ± 34.72 | 41.95 ± 53.81 | 22.38 ± 47.65 | 51.12 ± 31.58 |
| Minimum to maximum value | -63.6 to 100.0 | -204.0 to 100.0 | -91.7 to 100.0 | -16.7 to 100.0 |
| Median | 51.32 | 43.75 | 29.71 | 50.0 |
| Interquartile range | 35.71 to 70.03 | 18.18 to 80.00 | -5.57 to 55.64 | 27.78 to 72.73 |
| 95% confidence interval | 36.80 to 57.91 | 25.78 to 58.11 | 7.89 to 36.87 | 41.63 to 60.60 |

| | | | | |
|---|---|---|---|---|
| Unit (%) | | | | |

### Test Example 2

In order to evaluate the therapeutic effect of the external preparation of the present invention against acne vulgaris, a randomized, parallel-group comparison test was conducted for acne vulgaris patients.

### (1) Subject Patients

Patients, aged 13 or over and below 50, involving inflammatory lesions of 11 or more and 40 or less on face were chosen as subjects.

Individuals that fell into any of the following categories were excluded even if the foregoing conditions were met.
1) Patients involving complications on face that are considered to affect the evaluation of the test drug (e.g., acne other than acne vulgaris, and rosacea)
2) Patients complicated by a skin disease (e.g., atopic dermatitis) that may cause a facial lesion during the test
3) Patients showing visible changes occurring in acne symptoms due to menstrual cycle to such an extent that the efficacy evaluation will be affected
4) Patients who were internally administered with a retinoid or a medicinal agent that acts like a retinoid, or who had a retinoid or such a medicinal agent externally applied to face within 12 weeks before the start of treatment
5) Patients who used a systemically administered drug as an antibiotic for a total of 14 days or longer between 12 weeks before the start of treatment and 4 weeks before the start of treatment
6) Patients who had any of the following treatments within 4 weeks before the start of treatment
   - Treatments with systemically administered drugs, for example, therapeutic drugs for acne (excluding vitamin B2 and B6), steroids, and antibiotics, that are considered to affect the efficacy evaluation of the test drug
   - A chemical peel, a laser therapy or a phototherapy, acne extraction and popping, and other facial treatments that are intended to treat acne.
7) Patients who had any of the following treatments or procedures within 2 weeks before the start of treatment
   - Use of a locally administered drug to face, or facial esthetic procedures
   - Use of quasi drugs or cosmetics (e.g., facial scrub) for caring of facial acne
8) Patients who participated in other test within 4 months (120 days) before the start of treatment
9) Patients who have taken part in the observation period of this test
10) Patients with a history of hypersensitivity to synthetic antibiotics of the quinolone family, dermal hypersensitivity to external preparations, or photosensitivity
11) Patients suspected of serious complications through an interview
12) Pregnant patients, and patients wishing to be pregnant or who will be breast feeding during treatment
13) Patients who used forbidden combination drugs or received forbidden combination treatments during the observation period
14) Patients with less than 70% adherence to the test drug of morning or night during the observation period
15) Patients with a percentage reduction of 30% or more in the number of inflammatory lesions during the observation period
16) Patients who were determined to be inappropriate by a physician conducting the test.

### (2) Test Method

The control drug was administered over an observation period of 2 weeks, in the morning and at night. After the observation period, the subject patients were divided into test drug-administered groups (a once daily administered group, and a twice daily administered group), and a control drug-administered group. The test drug or the control drug was administered over a treatment period of 12 weeks, in the morning and at night.

The preparation prepared in Preparation Example 4 was used as the test drug. A lotion that did not contain compound A was used as the control drug.

For the once daily test drug-administered group, the control drug was administered in the morning, and the test drug was administered at night.

The face was inspected every two weeks after the treatment was started, and the number of inflammatory lesions was counted.

Here, inflammatory lesion means conditions involving an erythematous papule (a reddish elevated papula measuring no larger than 1 cm in diameter and matching hair follicles), or a pustule (involving occasional tenderness, and including a lesion involving a yellow pus at the top of an erythematous papule, and a lesion appearing yellowish white in color as a whole).

### (3) Evaluation Method

### 1) Main Evaluation Item

The following analysis was conducted with regard to the percentage reduction of the number of inflammatory lesions at the final evaluation time.

Summary statistics, and a 95% confidence interval were calculated for each administered group. A paired comparison was made between the control drug-administered group and the test drug-administered groups (twice daily group, and once daily group). Because a blind review showed outliners in the percentage reduction of the number of inflammatory lesions at the final evaluation time, separate-ranking Steel's test was conducted.

The Hodges-Lehmann estimator was calculated afterward for the difference between the median values of the control drug-administered group and the test drug-administered groups (twice daily group, and once daily group), along with a 95% confidence interval.

### 2) Sub Evaluation Item

From the median value data, time-course changes were calculated for the number of inflammatory lesions in the treatment period.

### (4) Evaluation Result

As shown in Table 6, there was a statistically significant difference in the percentage reduction of the number of inflammatory lesions at the final evaluation time between the test drug-administered groups (twice daily group, and once daily group) and the control drug-administered group (separate-ranking Steel's test; twice daily test-drug-administered group: p = 0.0003, once daily test drug-administered group: p = 0.0004).

As shown in FIG. 1, the number of inflammatory lesions decreased over time from week 2 to week 12 in all administered groups. The number of inflammatory lesions observed in the twice daily test drug-administered group and in the once daily test drug-administered group after week 4 was smaller than that observed in the control drug-administered group.

**[Table 6]**

| Administered group | Twice daily test drug-administered group | Once daily test drug-administered group | Control drug-administered group |
|---|---|---|---|
| Number of subjects | 112 | 113 | 112 |
| Mean (standard deviation) | 56.81 (43.91) | 56.96 (39.54) | 42.49 (37.54) |
| Median (minimum to maximum value) | 68.59 (-210.7 to 100.0) | 66.67 (-152.0 to 100.0) | 50.00 (-95.0 to 100.0) |
| Interquartile range | 43.43 to 81.53 | 40.00 to 82.14 | 22.54 to 68.90 |
| 95% confidence interval | 48.59 to 65.03 | 49.59 to 64.33 | 35.46 to 49.52 |
| Comparison between groups* (vs. control drug-administered group) | *P* = 0.0003 | *P* = 0.0004 | - |
| Median difference between groups† (95% confidence interval) | 15.08 (7.19 to 23.00) | 15.14 (7.41 to 23.08) | - |

| | | | |
|---|---|---|---|
| Unit (%) *: Separate ranking Steel's test †: Median difference between groups; Hodges-Lehmann estimator for median difference | | | |

### Test Example 3

In order to evaluate the therapeutic effect of the external preparation of the present invention against acne vulgaris, a randomized, parallel-group comparison test was conducted for acne vulgaris patients.

### (1) Subject Patients

Patients, aged 13 or over and below 50, involving inflammatory lesions of 11 or more and 40 or less on face were chosen as subjects.

Individuals that fell into any of the following categories were excluded even if the foregoing conditions were met.
1) Patients involving complications on face that are considered to affect the evaluation of the test drug (e.g., acne other than acne vulgaris, and rosacea)
2) Patients who are not suited for a bacteriological test (collection of the content of inflammatory lesion)
3) Patients complicated by a skin disease (e.g., atopic dermatitis) that may cause a facial lesion during the test
4) Patients showing visible changes occurring in acne symptoms due to menstrual cycle to such an extent that the efficacy evaluation will be affected
5) Patients who were internally administered with a retinoid or a medicinal agent that acts like a retinoid, or who had a retinoid or such a medicinal agent externally applied to face within 12 weeks before the start of treatment
6) Patients who used a systemically administered drug of an antibiotic for a total of 14 days or longer between 12 weeks before the start of treatment and 4 weeks before the start of treatment
7) Patients who had any of the following treatments within 4 weeks before the start of treatment
   - Treatments with systemically administered drugs, for example, therapeutic drugs for acne (excluding vitamin B2 and B6), steroids, and antibiotics, that are considered to affect the efficacy evaluation of the test drug
   - A chemical peel, a laser therapy or a phototherapy, acne extraction and popping, and other facial treatments that are intended to treat acne
8) Patients who had any of the following treatments or procedures within 2 weeks before the start of treatment
   - Facial treatments with locally administered drugs, for example, therapeutic drugs for acne, steroids, and antibiotics, that are considered to affect the efficacy evaluation of the test drug
   - Facial esthetic procedures
   - Use of quasi drugs or cosmetics (e.g., azelaic acid, benzoyl peroxide, and facial scrub) for caring of facial acne
9) Patients who participated in other test within 4 months (120 days) before the start of treatment
10) Patients who have participated in a test using an external preparation containing compound A
11) Patients who have participated in the same test
12) Patients with a history of hypersensitivity to synthetic antibiotics of the quinolone family, dermal hypersensitivity to external preparations, or photosensitivity
13) Patients suspected of serious complications (including systemic diseases) that make the patients inappropriate for participating in the test
14) Pregnant patients, and patients wishing to be pregnant or who will be breast feeding during treatment
15) Patients who used forbidden combination drugs or received forbidden combination treatments during the observation period
16) Patients with less than 70% adherence to the test drug of morning or night during the observation period
17) Patients with a percentage reduction of 30% or more in the number of inflammatory lesions during the observation period
18) Patients who were determined to be inappropriate by a physician conducting the test.

### (2) Test Method

The control drug was administered over an observation period of 2 weeks, in the morning and at night.

After the observation period, the subject patients were divided into a test drug-administered group, a comparative drug-administered group, and a control drug-administered group. A test drug, a comparative drug, or a control drug was administered over a treatment period of 12 weeks, in the morning and at night.

The preparation prepared in Preparation Example 4 was used as the test drug. A 1% nadifloxacin lotion (a commercially available product, twice daily) was used as the comparative drug. A lotion that did not contain compound A was used as the control drug.

For the test drug-administered group, the control drug was administered in the morning, and the test drug was administered at night.

The face was inspected every two weeks after the treatment was started, and the number of inflammatory lesions was counted.

Here, inflammatory lesion means conditions involving an erythematous papule (a reddish elevated papula measuring no larger than 1 cm in diameter and matching hair follicles), or a pustule (involving occasional tenderness, and including a lesion involving a yellow pus at the top of an erythematous papule, and a lesion appearing yellowish white in color as a whole).

### (3) Evaluation Method

### 1) Main Evaluation Items

The following analysis was conducted with regard to the percentage reduction of the number of inflammatory lesions at the final evaluation time.

Summary statistics were calculated for each administered group. A paired comparison (5% significance level on both sides) was made between the test drug-administered group and the control drug-administered group using a two-sample Wilcoxon test to assess superiority. If the assessment was positive for superiority, non-inferiority was assessed by determining whether the lower limit of the 95% confidence interval of the Hodges-Lehmann estimator for the difference between the median values of the test drug-administered group and the comparative drug-administered group was above the non-inferiority limit of -10.1%.

The non-inferiority limit was set from the result of Test Example 1. Specifically, the non-inferiority limit of 10.1% is approximately a half value of the difference, 20.29%, in the percentage reduction of the number of inflammatory lesions at the final evaluation time between the comparative drug-administered group and the control drug-administered group (median values of 50.00% and 29.71%, respectively)

### 2) Sub Evaluation Item

From the median value data, time-course changes were calculated for the number of inflammatory lesions in the treatment period.

### (4) Evaluation Result

As shown in Table 7, the percentage reduction (median value) of the number of inflammatory lesions at the final evaluation time was 54.77 % for the test drug-administered group, 53.59 % for the comparative drug-administered group, and 41.67% for the control drug-administered group.

A paired comparison between the test drug-administered group and the control drug-administered group showed a significance difference (p < 0.001, two-sample Wilcoxon test), confirming that the test drug-administered group was superior to the control drug-administered group.

The Hodges-Lehmann estimator (95% confidence interval) for the difference in the median value of the percentage reduction between the test drug-administered group and the comparative drug-administered group was 0.00% (-7.14% to 6.35%), and the lower limit of the 95% confidence interval was above the non-inferiority limit of - 10.1%, confirming the non-inferiority of the test drug-administered group against the comparative drug-administered group.

As shown in FIG. 2, in the test drug-administered group and the comparative drug-administered group, the percentage reduction of the number of inflammatory lesions increased with time from week 2 to week 12 after the start of treatment in their evaluation periods, and the pattern was the same between these two groups.

From the estimator (95% confidence interval) of the median value of the percentage reduction of the number of inflammatory lesions between the test drug-administered group and the control drug-administered group, a comparison with the control drug-administered group showed a significant difference between the test drug-administered group and the control drug-administered group after 2 weeks from the start of treatment.

**[Table 7]**

| Administered group (n) | First day of treatment (number of inflammatory lesions) | At final evaluation time (number of inflammatory lesions) | Reduction (number of inflammatory lesions) | Percentage reduction (%) |
|---|---|---|---|---|
| Test drug-administered group (n = 204) | 16.0 (13.0 to 21.0) | 7.0 (4.0 to 12.0) | 9.0 (5.0 to 13.0) | 54.77 (29.92 to 75.74) |
| Comparative drug-administered group (n = 198) | 15.0 (13.0 to 20.0) | 7.5 (3.0 to 12.0) | 9.0 (4.0 to 12.0) | 53.59 (26.32 to 81.82) |
| Difference from test drug-administered group | - | - | 0.0(-1.0 to 2.0) | 0.00 (-7.14 to 6.35) |
| Step 2* | | | | Lower limit of confidence interval > non-inferiority limit of -10.1% |
| Control drug-administered group (n = 97) | 15.0 (13.0 to 19.0) | 9.0 (6.0 to 16.0) | 6.0 (0.0 to 10.0) | 41.67 (0.00 to 66.67) |
| Difference from test drug-administered group | - | - | 3.0 (2.0 to 5.0) | 15.85 (6.67 to 25.00) |
| Step 1* | | | | *P* = 0.0007 (two-sample Wilcoxon test) |

| | | | | |
|---|---|---|---|---|
| First day of treatment (number of inflammatory lesions), At final evaluation time (number of inflammatory lesions), Reduction (number of inflammatory lesions), Percentage reduction (%): Median (interquartile range) Difference from test drug-administered group: Hodges-Lehmann estimator (95% confidence interval) | | | | |

### Test Example 4

In order to evaluate the therapeutic effect of the external preparation of the present invention against a superficial infection of the skin, a non-blind test was conducted for patients suffering from folliculitis or sycosis, a typical superficial infection of the skin.

### (1) Subject Patients

Patients, aged 13 or over, involving folliculitis or sycosis with moderate or more severe symptoms of all of redness (newly occurring redness, and excluding a residual erythema dark red in color), swelling (acute inflammatory swelling, and excluding a post-inflammation fibrosis), and erythematous papule and pustule, and having multiple lesions at the evaluation site were chosen as subjects.

Individuals that fell into any of the following categories were excluded even if the foregoing conditions were met.
1) Patients who are not suited for a bacteriological test (collection of the content of an erythematous papule or a pustule)
2) Patients with other skin diseases or systemic diseases that are considered to affect the evaluation of the test drug
3) Patients who used a systemically administered drug of an antibiotic within 4 weeks before the start of treatment
4) Patients who used a locally administered drug of an antibiotic to a lesion at a site of efficacy evaluation within 2 weeks before the start of treatment
5) Patients with a history of hypersensitivity to synthetic antibiotics of the quinolone family, dermal hypersensitivity to external preparations, or photosensitivity
6) Patients suspected of serious complications through an interview
7) Pregnant or breast feeding patients, or patients not wishing to take birth control measures during the test
8) Patients who participated in a test within 4 months (120 days) before the start of treatment
9) Patients who have participated in a test using an external preparation containing compound A
10) Patients who have participated in the same test
11) Patients who were determined to be inappropriate by a physician conducting the test.

### (2) Test Method

On the first day of treatment, an evaluation site was specified from regions where moderate to more severe symptoms of all of redness, swelling, and erythematous papule and pustule were observed.

An appropriate amount of the preparation prepared in Preparation Example 4 was applied with a clean finger tip over the whole lesion area (including an area outside of the evaluation site), once daily (night) for 7 days.

After the treatment was started, the evaluation site was inspected on day 3 and day 7, and the efficacy was determined from the observed skin conditions (redness, swelling, and erythematous papule and pustule) by scoring the result for each item.

Skin conditions were scored using the criteria shown in Table 8. Efficacy was determined according to the criteria shown in Table 9.

### (3) Evaluation Method

The efficacy rate, and the 95% confidence interval of the efficacy rate were calculated at the final evaluation time from the scores of the skin conditions observed at the evaluation site.

### (4) Evaluation Result

The efficacy rate was 70.0%, as shown in Table 10.

**[Table 8]**

| Observation | Score | Severity | Folliculitis | Sycosis |
|---|---|---|---|---|
| Redness | 0 | - | Absent | Absent |
| | 1 | Mild | Slight redness | Slight redness |
| | 2 | Moderate | Clearly different shade from surrounding area, and the boundary is clearly visible | Clearly different shade from surrounding area, and the boundary is clearly visible |
| | 3 | Severe | Darker shade with sensation of heat | Darker shade with sensation of heat |
| Swelling | 0 | - | Absent | Absent |
| | 1 | Mind | Slight elevation | Slight elevation |
| | 2 | Moderate | Clear elevation, and the boundary from surrounding area is clearly visible | Clear elevation, and the boundary from surrounding area is clearly visible |
| | 3 | Severe | Marked elevation, and the skin feels a little tense when pressed | Marked elevation, and the skin feels a little tense when pressed |
| Erythematous papule, pustule | 0 | - | Absent | Absent |
| | 1 | Mild | Small-sized erythematous papule or pustule (2 mm or less) | Small numbers of small-sized erythematous papule or pustule (2 mm or less) are visible |
| | 2 | Moderate | Middle-sized erythematous papule or pustule (3 to 4 mm) | Large numbers of small-sized erythematous papule or pustule (2 mm or less), and small numbers of middle-sized erythematous papule or pustule (3 to 4 mm) are visible |
| | 3 | Severe | Moderately large erythematous papule or pustule (5 mm or more) | Multiple occurrences of moderately large erythematous papule or pustule (5 mm or more) |

**[Table 9]**

| Determination | Criteria |
|---|---|
| Highly effective | - Scores for all items were 0 on the last day of evaluation |
| | - Score improved by 2 or more points for at least two items after 3 days, and the total score on the last day of evaluation was improved by 1 or more points from the total score after 3 days |
| Effective | Score improved by 2 or more points for at least two items by the last day of evaluation |
| Moderately effective | Total score improved by 1 or more points by the last day of evaluation |
| Ineffective | Total score remained unchanged, or worsened by 1 or more points by the last day of evaluation |

**[Table 10]**

| Group analyzed for effectiveness | Criteria for effectiveness determination | | | | Efficacy rate at final evaluation time | |
|---|---|---|---|---|---|---|
| Number of subjects | Highly effective | Effective | Moderately effective | Ineffective | Highly effective + effective | Efficacy rate (95% confidence interval) |
| 40 | 12 (30.0) | 16 (40.0) | 12 (30.0) | 0 | 28 | 70.0 (53.5 to 83.4) |

| | | | | | | |
|---|---|---|---|---|---|---|
| Efficacy rate: Percentage of "highly effective" or "effective" Number of subjects (%) | | | | | | |

## Claims

1. An external preparation that comprises 1-cyclopropyl-8-methyl-7-[5-methyl-6-(methylamino)-3-pyridyl]-4-oxo-1,4-dihydro-3-quinolinecarboxylic acid and/or a pharmaceutically acceptable salt thereof as an active ingredient, and that exerts a therapeutic and/or preventive effect against a dermatological infection upon being administered to a human patient once daily.

2. The external preparation according to claim 1, wherein the dermatological infection is an uncomplicated skin and soft tissue infection, a complicated skin and soft tissue infection, or acne involving suppurative inflammation.

3. The external preparation according to claim 2, wherein the uncomplicated skin and soft tissue infection is a superficial infection of the skin, or a deep skin infection.

4. The external preparation according to claim 3, wherein the superficial infection of the skin is folliculitis, sycosis, purulent periporitis, or contagious impetigo.

5. The external preparation according to claim 2, wherein the acne involving suppurative inflammation is acne vulgaris, neonatal acne, or acne conglobata.

6. The external preparation according to any one of claims 1 to 5, which has a form of an ointment, a gel, a cream, an emulsion, an adhesive tape, or a lotion.

7. The external preparation according to any one of claims 1 to 5, which has a form of a lotion.

8. The external preparation according to claim 7, which contains a lower alcohol, a water-soluble polymer, and a polyalcohol, and has a pH of 9 to 12.

9. The external preparation according to claim 8, wherein the lower alcohol is ethanol or isopropanol.

10. The external preparation according to claim 8, wherein the water-soluble polymer is a water-soluble cellulose derivative.

11. The external preparation according to claim 10, wherein the water-soluble cellulose derivative is hydroxyethyl cellulose.

12. The external preparation according to claim 8, wherein the polyalcohol is 1,3-butylene glycol.

13. The external preparation according to claim 8, wherein the lower alcohol is ethanol, the water-soluble polymer is a water-soluble cellulose derivative, and the polyalcohol is 1,3-butylene glycol.

14. The external preparation according to claim 13, wherein the ethanol is contained in a content of 1 to 20 weight%.
